# EUROPÄISCHE PATENTANMELDUNG

(11) **EP 3 409 315 A1**
(43) Veröffentlichungstag der Anmeldung: **05.12.2018**
(21) Anmeldenummer: 17174196.0
(22) Anmeldetag: 02.06.2017
(51) Int. Cl.: A61M 15/00, A61M 16/20

(54) **INHALATIONSSYSTEM**

(71) Anmelder: R. Cegla GmbH & Co. KG, 56410 Montabaur (DE)
(72) Erfinder: Hartmann Cegla, Ulrich, 56410 Montabaur (DE)
(74) Vertreter: Engelhardt, Volker

(57) **Zusammenfassung**

Inhalationssystem (1) zur Einatmung eines Dosieraerosols (6') bestehend aus:
- einem das Aerosol ausgebenden Dosiergerät (6),
- einem Behälter (2), in dem durch ein oder mehrere Ventile (8) voneinander getrennte und beim Einatmen miteinander verbundenen Kammern (4, 5) vorgesehen sind,
- wobei das Ventil aus einem elastisch verformenden Werkstoff gebildet ist, und einen ringförmig umlaufenden Bund (21) aufweist, der an dem Behälter fixiert ist,
- wobei in das Ventil mindestens eine schlitzartige Einkerbung (22) eingearbeitet ist, die als von innen nach außen verlaufende Spirale ausgebildet ist,
- wobei beim Einatmen das Ventil auf das diese durchströmende Aerosol eine Strömungsbarriere ausübt, durch die das Aerosol in eine lineare Bewegung und eine spiralförmige Strömungsrichtung (11) entlang zu der und um die Längsachse (3) des Behälters in eine dreidimensionale Spirale geleitet ist.

## Beschreibung

Die Erfindung bezieht sich auf eine Inhalationssystem zur Einatmung eines Dosieraerosols nach dem Oberbegriff des Patentanspruches 1.

Eine solche Inhalationsanordnung ist beispielsweise aus der EP 2 678 060 B1 bekannt geworden. Derartige Inhalationsanordnungen bestehen aus einem Behälter, der zwei durch ein Ventil miteinander verbundene Kammern aufweist. Das Ventil ist bei dem vorgenannten Stand der Technik als Membrane ausgestaltet, in die zwei senkrecht zueinander verlaufende Schlitze eingearbeitet sind, die sich während des Ausatmungsvorganges verschließen; wohingegen beim Einatmen die derart gebildeten Kreuzschlitze geöffnet sind, so dass aus der ersten Kammer das dort eingedrückte Aerosol in die zweite Kammer strömen kann. Das Aerosol wird mittels eines Dosiergerätes eingebracht, das an einer Einlassöffnung angeordnet ist, die in die erste Kammer mündet.

Aufgrund der Volumenvergrößerung zwischen dem Dosiergerät und der ersten Kammer verteilt sich das Aerosol in der ersten Kammer gleichmäßig und kann von einem Patienten durch einen üblichen Einatmungsvorgang eingesaugt werden. Während des Einatmungsvorganges entsteht in der zweiten Kammer ein Unterdruck, durch den das Ventil zwischen der ersten und zweiten Kammer geöffnet ist, so dass das Aerosol aus der ersten in die zweite und von dieser über eine erste Durchgangsöffnung in ein Mundstück einströmt, das beispielsweise mit dem Mund eines Patienten verbunden ist.

Während des Ausatmungsvorganges ist die zweite Kammer von der ersten Kammer durch Verschließen des Ventils zu trennen, so dass keine Atemluft aus der zweiten Kammer in die mit Aerosol gefüllte erste Kammer einströmen kann. Vielmehr soll die ausgeartete Luft des Patienten aus der zweiten Kammer mittels einer zweiten Durchgangsöffnung in die Umgebung entweichen.

Als nachteilig hat sich bei diesem Stand der Technik herausgestellt, dass zwar die Handhabung der Inhalationsanordnung einfach und hygienisch bewerkstelligt werden kann, da die einzelnen die Inhalationsanordnung bildenden Bauteile sterilisiert werden können, jedoch entsteht während des Einatmungsvorganges eine nahezu lineare, zylinderförmige Luftströmung, denn die in die Membrane eingearbeiteten Kreuzschlitze verlaufen senkrecht zueinander, so dass diese nahezu keinen Strömungseinfluss auf die von der ersten in die zweite Kammer strömenden Aerosol ausüben. Folglich ist auch die Vermengung des in der zweiten Kammer eingesaugten Aerosols mit der dort vorhandenen Luft aus der Umgebung gering oder existiert überhaupt nicht, da im Wesentlichen von der Membrane eine zylinderförmige Aerosol-Strömung gebildet ist, die sich mit der in der zweiten Kammer eingesaugten Luft aus der Umgebung nahezu nicht vermengt. Folglich gelangt hoch angereichertes Aerosol in den Mund des Patienten, dessen Wirkung in Bezug auf die eingeatmete Menge des Aerosols gering ist, da keine optimale Verteilung des Aerosols in den Atemwegen stattfindet und diese oftmals die Bronchien oder Lungen nicht in ausreichender Menge erreichen, da das Aerosol bereits an den Schleimhäuten des Mundes oder Rachen abgelagert ist.

Es ist daher Aufgabe der Erfindung eine Inhalationsanordnung der eingangs genannten Gattung derart weiterzubilden, dass zum einen die einfache und hygienische Anwendung der Inhalationsanordnung gewährleistet ist, und dass zum anderen die Aerosol-Strömung während des Einatmungsvorganges in eine lineare und rotierende Bewegung geleitet ist, die sich im Wesentlichen entlang der und um die Längsachse des Behälters der Inhalationsanordnung bewegt und eine derart geschaffene Rotationsbewegung des Aerosols in den Mund-, Rachen-und Lungenbereich des Patienten gelangt, um das mittransportierte Aerosol möglichst großflächig und gleichmäßig auf den zu behandelnden Bereich des Patienten zu transportieren.

Diese Aufgaben sind erfindungsgemäß durch die Merkmale des kennzeichnen Teils von Patentanspruch 1 gelöst.

Weitere vorteilhafte Weiterbildungen der Erfindung ergeben sich aus den Unteransprüchen.

Dadurch, dass das Ventil aus einem elastisch verformenden Werkstoff, vorzugsweise aus Silikon, gebildet ist, dass das Ventil einen ringförmig umlaufenden Bund aufweist, der an dem Behälter fixiert ist, dass in das Ventil eine schlitzartige Einkerbung eingearbeitet ist, die als von außen nach innen sich verringernde Spirale ausgebildet ist, und dass beim Einatmen die Einkerbungen des Ventils auf das durchströmende Aerosol eine Strömungsbarriere ausübt, durch die das Aerosol in eine lineare und rotierende Bewegung entlang zu der und um die Längsachse des Behälters geschaffen ist, ist erreicht, dass das in die zweite Kammer eingesaugte Aerosol durch die Ausgestaltung des Ventils eine lineare Vorwärtsbewegung und eine Rotation um die Längsachse des Behälters erfahren, die zudem unterschiedliche Durchmesser aufweisen. Das Ventil besteht nämlich aus einer spiralförmigen von innen nach außen verlaufenden Struktur, die während des Einatmungsvorganges als Strömungsbarriere auf das eingeatmete Aerosol einwirkt und dieses demnach in die entsprechende Richtung umlenkt, wodurch eine dreidimensionale Strömungskontur des Aerosols bei jedem Einatmungszyklus entsteht. Eine solche Aerosol-Struktur lagert sich während des Einatmungsvorganges an völlig unterschiedlichen Positionen im Mund-, Rachen- bzw. Lungenbereich des Patienten ab, wodurch eine gleichmäßige und in allen medizinisch notwendigen Bereichen des Menschen eindringende Behandlung mittels des Aerosols sichergestellt ist.

Aufgrund der Ausgestaltung des Ventils in einen ringförmigen umlaufenden Bund ist gewährleistet, dass das Ventil positionsgenau und lageorientiert an dem Behälter fixiert werden kann. Da der Behälter zweiteilig ausgestaltet ist, kann das Ventil schnell ausgewechselt werden, um diese beispielsweise in einem Heißwasserbad oder einer Mikrowelle zu sterilisieren bzw. zu reinigen.

Da das Ventil aus einem elastisch verformbaren Werkstoff, vorzugsweise aus Silikon, hergestellt ist, ist zum einen erreicht, dass die Vorspannkraft des Ventils dem Überdruck in der ersten Kammer entgegenwirkt, so dass das Aerosol aus der ersten Kammer in die zweiten Kammer ausschließlich dann überführt ist, wenn der Patient durch eine Einatmung in der zweiten Kammer einen Unterdruck schafft, durch den gemeinsam mit dem Überdruck in der ersten Kammer das Ventil spiralförmig angehoben ist, und zum anderen, dass das Ventil in jeder beliebigen Position eine hohe Formstabilität aufweist, so dass dieses nicht abknickt oder durchschnappt.

In der Zeichnung ist ein erfindungsgemäßes Ausführungsbeispiel einer Inhalationsanordnung dargestellt, die nachfolgend näher erläutert ist. Im Einzelnen zeigt:
- Figur 1: ein Inhalationssystem bestehend aus einem zweiteiligen Behälter, in dem ein Ventil gehalten ist, durch das der Behälter in zwei Kammern unterteilt ist, aus einem Dosiergerät, das in die erste Kammer des Behälters einmündet, wobei in der zweiten Kammer zwei Durchgangsöffnungen zum Einatmen und zum Ausatmen vorgesehen sind, in Seitenansicht,
- Figur 2a: das Inhalationssystem gemäß Figur 1 in vergrößerter Darstellung während des Einatmungsvorganges und
- Figur 2b: das Inhalationssystem gemäß Figur 1 während des Ausatmungsvorganges.

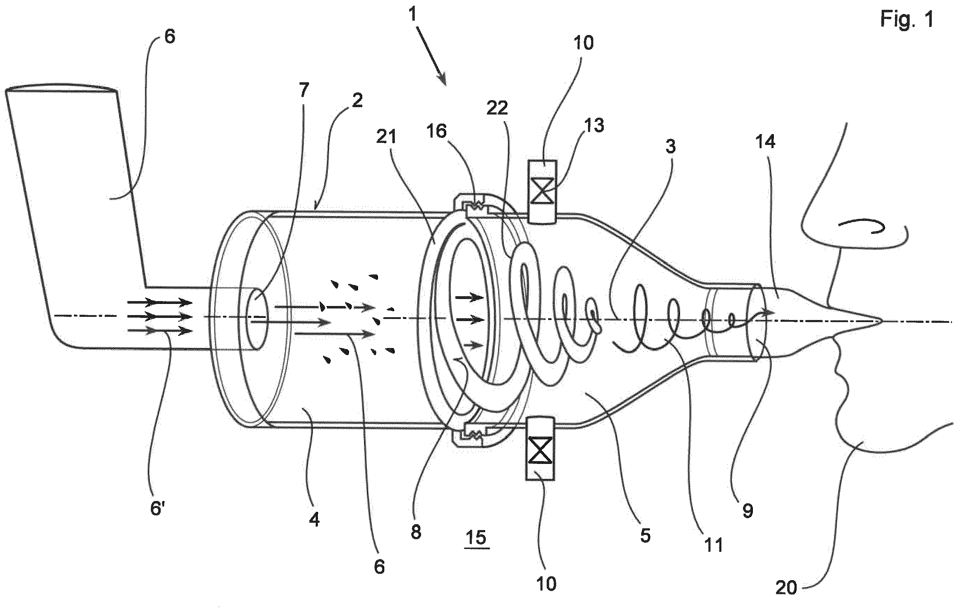

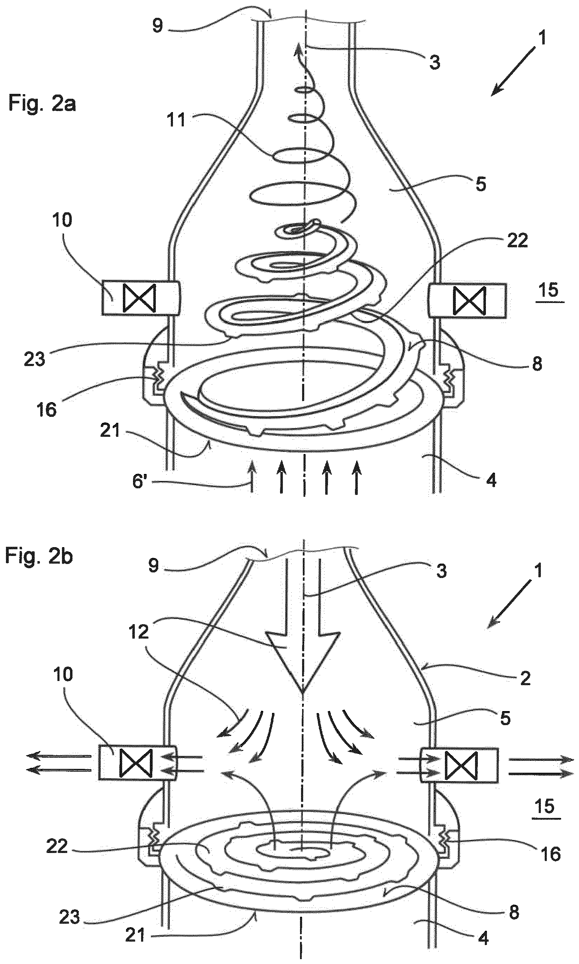

In den Figuren 1 und 2a ist ein Inhalationssystem 1 zu entnehmen, das dazu dient, ein von einem Dosiergerät 6 abgegebenes Aerosol 6' an einen Menschen 20 zur Therapie von Atemwegserkrankungen zu transportieren. Insbesondere ist zu gewährleisten, dass das Dosieraerosol 6' möglichst gleichmäßig und optimal dosiert in den Bronchialbereich des Patienten 20 gelangt und sich an den zu behandelnden Stellen des Menschen 20 gleichmäßig ablagern kann.

Das Inhalationssystem 1 besteht aus einem Behälter 2, dessen Längsachse mit der Bezugsziffer 3 versehen ist. Der Behälter 2 weist zwei Teile auf, die miteinander lösbar verbunden sind. Der Übergangsbereich zwischen den beiden Teilen des Behälters 2 wird als Trendebene definiert, in die ein nachfolgend näher erläutertes Ventil 8 lageorientiert eingesetzt und arretiert ist. Folglich entstehen im Inneren des Behälters 2 zwei Kammern 4 und 5, die durch das Ventil 8 voneinander getrennt sind, jedoch miteinander kommunizieren können, wenn das Ventil 8 geöffnet ist. Sobald das Ventil 8 geschlossen ist, sind die beiden Kammern 4 und 5 luftdicht voneinander getrennt.

In die erste Kammer 4 ist im Bereich der Längsachse 3 des Behälters 2 eine Einlassöffnung 7 eingearbeitet, in die das Dosiergerät 6 eingesetzt ist. Durch Betätigen des Dosiergerätes 6 gelangt parallel zu der Längsachse 3 in den von der ersten Kammer 4 gebildeten Raum das Aerosol 6' und verteilt sich somit in der ersten Kammer 4.

In die zweite Kammer 5 sind zwei Durchgangsöffnungen 9 und 10 vorgesehen. Die erste Durchgangsöffnung 9 liegt fluchtend zu der Längsachse 3 des Behälters 2 und kann beispielsweise mit einem Mundstück 14 versehen sein, das in den Mund des Patienten 20 zum Ein- und Ausatmen einsetzbar ist. Die Durchgangsöffnungen 10 befinden sich am Fuss der Spirale 21 und öffnen sich beim Ausatmen bzw. verschließen sich bei der Einatmung.

Insbesondere aus Figur 2a ist ersichtlich, dass das Ventil 8 aus einem ringförmig umlaufenden Bund 21 gebildet ist, der zwischen den beiden Teilen des Behälters 2 im Bereich des Gewindes 16 eingesetzt ist und somit zwischen den beiden Teilen des Behälters 2 lageorientiert fixiert ist. Das Ventil 8 ist eben ausgestaltet und verläuft senkrecht zu der Längsachse 3 des Behälters 2.

Durch Betätigen des Dosiergerätes 6 gelangt Aerosol 6' in die erste Kammer 4 und verteilt sich in dieser möglichst gleichmäßig. Folglich entsteht in der ersten Kammer 4 ein Überdruck. Das Ventil 8 ist aus einem elastisch verformbaren Werkstoff, vorzugsweise aus Silikon, hergestellt. Dieser Werkstoff weist eine derart hoch bemessene Vorspannkraft auf, dass durch diese der in der ersten Kammer 4 vorherrschende Überdruck des Aerosol 6' gehalten ist. Demnach ist das Ventil 8 luftdicht verschlossen und das in der ersten Kammer 4 vorhandene Aerosol 6' kann aus dieser nicht in die zweite Kammer 5 austreten. Erst wenn durch den Patienten 20 durch Einatmen in der zweiten Kammer 5 ein Unterdruck erzeugt ist, öffnet sich das Ventil 8, so dass das Aerosol 6' aus der ersten Kammer 4 durch das Ventil 8 in die zweite Kammer 5 einströmt.

Das Ventil 8 weist eine sich von außen nach innen verjüngend spiralförmige Einkerbung 22 auf. Sobald demnach der Patient 20 einatmet - dies ist als Einatmungsvorgang bezeichnet - wird das Ventil 8 spiralförmig angehoben. Das Ventil 8 weist demnach in diesem Zustand eine umlaufende Strömungskontur auf, die eine dreidimensionale Form einnimmt. Die derart geschaffene dreidimensionale Struktur des Ventiles 8 steht als Strömungsbarriere dem eingesaugten Aerosol 6' entgegen, wodurch dieses in eine spiralförmige Drehung um die Längsachse 3 sowie gleichzeitig in eine lineare Zustellbewegung überführt ist, die in Richtung der ersten Durchgangsöffnung 9 strömen bzw. von dem Patienten 20 angesaugt ist. Die derart geschaffene Strömungsrichtung des eingeatmeten Aerosol 6' ist mit der Bezugsziffer 11 gekennzeichnet. Da sämtliche Luftströmungen parallel zu der Längsachse 3 und um diese herum verlaufen, gelangt eine derart geschaffene Aerosol-Strömung 11 in den Mund-, Rachen-und Lungenbereich des Patienten 20, so dass sich die Aerosol 6'-Strömungen gleichmäßig in den zu behandelnden Bereichen des Patienten 20 ablagern können. Aufgrund der geometrischen Ausgestaltung des Ventiles 8, durch dessen spiralförmige Einkerbung 22 entsteht nämlich eine Aerosol-Strömung, da für jede Wendel der spiralförmigen Einkerbung 22 ein entsprechend groß bemessener Durchmesser vorliegt.

Der Werkstoff des Ventiles 8 ist zwar aus einem elastisch verformbaren Werkstoff, vorzugsweise Silikon, hergestellt, jedoch weist dieser Werkstoff eine gewisse Formstabilität auf, so dass das geöffnete Ventil 8 nicht wegknickt. Das Ventil 8 kann zudem aus dem Behälter 2 entfernt werden, um dieses in einem Heißwasserbad oder einer Mikrowelle zu reinigen. Sollte das Ventil 8 aufgrund intensiver Benutzung verschleißen, kann dieses ohne weiteres ausgewechselt bzw. ersetzt werden.

In Figur 2b ist der Ausatmungsvorgang des Patienten 20 und dessen Auswirkung auf das Ventil 8 dargestellt. Die Strömungsrichtung der ausgeatmeten Luft ist mit der Bezugsziffer 12 versehen, die durch das Mundstück 14 in die zweite Kammer 5 einströmt. Es ist unbedingt zu vermeiden, dass die ausgeatmete Luft des Patienten 20 in die erste Kammer 4 gelangt und sich dort mit dem eingepresst Aerosol 6' vermengt und es anfeuchtet. Daher ist es erforderlich, dass das Ventil 8 während des Ausatmungsvorganges des Patienten 20 luftdicht verschlossen ist. Die ausgeatmete Luft des Patienten 20 soll demnach aus der zweiten Kammer 5 über die zweite Durchgangsöffnung 10 in die Umgebung 15 entweichen. Das Ventil 8 verschließt in diesem Zustand die beiden Kammern 4 und 5 luftdicht voneinander.

Um ein Durchschnappen oder Verformen des Ventiles 8 während des Ausatmungsvorganges des Patienten 20 zu verhindern, sind im Bereich der Einkerbung 22 Anlageplatten 23 angeformt, die sich überlappend von innen nach außen auf dem jeweils benachbarten Band des Ventiles 8 abstützen. Durch die Anlageplatten 23 entsteht demnach eine schuppenartige Struktur, die der Überdruck in der zweiten Kammer 5 entgegensteht und somit eine formstabile und luftdichte Struktur des Ventiles 8 erreicht ist.

## Patentansprüche

1. Inhalationssystem (1) zur Einatmung eines Dosieraerosols (6') bestehend aus:
- einem das Aerosol (6') ausgebenden Dosiergerät (6),
- einem Behälter (2), in dem ein oder mehrere Ventile (8) voneinander getrennte und beim Einatmen miteinander verbundenen Kammern (4, 5) vorgesehen sind,
- wobei in die erste Kammer (4) eine Einlassöffnung (7) mündet, in die das Dosiergerät (6) eingesetzt ist,
- wobei die zweite Kammer (5) eine erste Durchgangsöffnung (9) aufweist, die mit den Atemöffnungen eines Menschen (20) zusammenwirkt,
- wobei die zweite Kammer (5) eine zweite Durchgangsöffnung (10) aufweist, die mit der Umgebung (15) verbunden ist und durch die beim Ausatmen ein Überdruck in der zweiten Kammer (5) in die Umgebung (15) entweichen kann,
**dadurch gekennzeichnet,**
- **dass** das Ventil (8) aus einem elastisch verformenden Werkstoff, vorzugsweise aus Silikon, gebildet ist, dass das Ventil (8) einen ringförmig umlaufenden Bund (21) aufweist, der an dem Behälter (2) fixiert ist,
- **dass** in das Ventil (8) mindestens eine schlitzartige Einkerbung (22) eingearbeitet ist, die als von innen nach außen verlaufende Spirale ausgebildet ist.
- **dass** beim Einatmen das Ventil (8) auf das diese durchströmende Aerosol (6') eine Strömungsbarriere ausübt, durch die das Aerosol (6') in eine lineare Bewegung und eine spiralförmige Strömungsrichtung (11) entlang zu der und um die Längsachse (3) des Behälters (2) in eine dreidimensionale Spirale geleitet ist.

2. Inhalationsanordnung nach Anspruch 1,
**dadurch gekennzeichnet,**
**dass** im Bereich der Einkerbungen (22) des Ventils (8) eine oder mehrere übergreifende Anlageplatten (23) vorgesehen sind, durch die das Ventil (8) beim Ausatmen formstabil und luftdicht verschlossen ist.

3. Inhalationsanordnung nach Anspruch 1 oder 2,
**dadurch gekennzeichnet,**
**dass** das Ventil (8) in einer Ebene angeordnet ist, die senkrecht zu der zu der Längsachse (3) des Behälters (2) verläuft.

4. Inhalationsanordnung nach Anspruch 1,
**dadurch gekennzeichnet,**
**dass** das Ventil (8) in Richtung der Längsachse (3) des Behälters (2) domförmig oder kugelförmig gekrümmt ist und dass in das Ventil (8) mindestens eine Einkerbung (22) eingearbeitet ist, die in Strömungsrichtung (11) ansteigend verläuft.

5. Inhalationsanordnung nach Anspruch 4,
**dadurch gekennzeichnet,**
**dass** im Bereich der Einkerbungen (22) eine oder mehrere Überstände vorgesehen sind, durch die die Einkerbungen (22) abgedeckt sind, und dass die Überstände in Bezug auf die Strömungsrichtung (11) des Aerosols (6') oberhalb der benachbarten Einkerbungen (22) verlaufen.

6. Inhalationsanordnung nach einem der vorgenannten Ansprüche,
**dadurch gekennzeichnet,**
**dass** in der ersten Kammer (4) ein von dem Dosiergerät (6) erzeugter Aerosol-Überdruck vorherrscht, dass die Vorspannung Ventils (8) derart bemessen ist, dass der in der ersten Kammer (4) vorliegende Überdruck in dieser eingeschlossen ist und dass ein durch das Einatmen erzeugter Überdruck in der zweiten Kammern (5) des Ventils (8) in Richtung der zweiten Kammer (5) anhebt.

7. Inhalationsanordnung nach einem der vorgenannten Ansprüche,
**dadurch gekennzeichnet,**
**dass** der Behälter (2) zweitteilig ausgestaltet ist, dass die beiden Teile des Behälters (2) mittels Schrauben, eines Gewindes (16) oder per Click-Verfahren miteinander lösbar verbunden sind, und dass der ringförmige Bund (21) das Ventil (8) in der Trennebene der beiden Teile des Behälters (2) befestigt ist oder anderweitig fixiert wird, so dass sich das Ventil (8) und der ringförmige Bund (21) festgesetzt sind.

8. Inhalationsanordnung nach einem der vorgenannten Ansprüche,
**dadurch gekennzeichnet,**
**dass** das Ende der Einkerbung (22) fluchtend zu der Längsachse (3) des Behälters (2) während des Ein- und Ausatmungsvorganges verläuft.
